# FASCICULE DE BREVET EUROPEEN

(11) **EP 1 362 049 B1**
(45) Date de publication et mention de la délivrance du brevet: **21.06.2006**
(21) Numéro de dépôt: 02704819.8
(22) Date de dépôt: 13.02.2002
(51) Int. Cl.: C07D 231/00

(54) **PYRAZOLO-TRIAZOLES ET UTILISATION DE CES COMPOSES POUR LA TEINTURE DE FIBRES KERATINIQUES**
PYRAZOLOTRIAZOLE UND DEREN VERWENDUNG ZUM FÄRBEN VON KERATINFASERN
PYRAZOLO-TRIAZOLES AND USE OF SAID COMPOUNDS FOR DYEING KERATINOUS FIBRES

(30) Priorité: 14.02.2001 FR 0102001
(43) Date de publication de la demande: 19.11.2003
(73) Titulaire: L'ORÉAL, 75008 Paris (FR)
(72) Inventeur: VIDAL, Laurent, F-75013 Paris (FR); GENARD, Sylvie, F-94130 Nogent sur Marne (FR)
(74) Mandataire: Fevrier, Murielle Françoise E.
(86) Numéro de dépôt international: PCT/FR2002/000552
(87) Numéro de publication internationale: WO 2002/064596

(56) Documents cités:
- EP-A- 0 923 929
- EP-A- 1 040 818
- WO-A-97/35551
- J. BAILEY: "Synthesis of +H-pyrazolo(3,2-c)-s-triazoles and derived azamethine dyes" JOURNAL OF THE CHEMICAL SOCIETY, PERKIN TRANSACTIONS 1., 1977, pages 2047-2052, XP002182052 CHEMICAL SOCIETY. LETCHWORTH., GB ISSN: 1472-7781 cité dans la demande

## Description

L'invention a pour objet de nouveaux pyrazolo-[3,2-c]-1,2,4-triazoles utiles pour la teinture par oxydation des fibres kératiniques, en particulier des cheveux humains, les compositions contenant ces composés ainsi que le procédé de teinture les mettant en oeuvre.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des composés pyrazolo-azole à titre de coupleurs pour la teinture de fibres kératiniques. Par exemple, les demandes de brevet EP 1 040 818 et FR 2 746 306 décrivent, des compositions pour la teinture des fibres kératiniques contenant de tels coupleurs. Ces compositions ne sont cependant pas totalement satisfaisantes, en particulier, elles ne sont pas totalement satisfaisantes au niveau de la pureté des nuances.

Le but de la présente invention est de fournir de nouvelles compositions tinctoriales ne présentant pas les inconvénients de celles de la technique antérieure. En particulier, le but de la présente invention est de fournir des compositions pour la teinture de fibres kératiniques par oxydation qui présentent des teintures puissantes, peu sélectives et particulièrement résistantes, capables d'engendrer des colorations intenses dans des nuances variées, et qui présentent une coloration qui évolue peu lors d'une application différée.

Ce but est atteint avec la présente invention qui a pour objet un nouveau pyrazolo-[3,2-c]-1,2,4-triazole de formule (I) ou l'un de ses sels d'addition avec un acide ou une base: 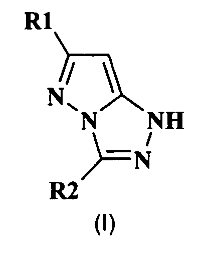 dans laquelle
R1 représente un groupe hydroxy ou un groupe alkyle en C1-C4, substitué par au moins un radical hydroxy, et
R2 représente un groupe aryle, par exemple phényle ou naphtyle, pouvant être substituté par au moins un radical choisi dans le groupe constitué par les radicaux halogène, nitro, cyano, alkyle, hydroxyle, alcoxy, amino, alkylamino, dialkylamino, acylamino, sulfonamido, alkylthio, alcoxycarbonyl, carboxy et sulfo.

Selon un mode de réalisation particulier, R1 est un groupe alkyle en C1-C4, substitués par 1 à 3 radicaux hydroxyle et R2 est un groupe phényle pouvant être substitué par 1 à 3 radicaux, de préférence 1 à 2 radicaux.

R1 est par exemple choisi parmi l'hydroxyméthyle, le 2-hydroxyéthyle, le 3-hydroxypropyle, le 1-hydroxyéthyle, le 1-hydroxypropyle, le 1-méthyl-2-hydroxyéthyle, le 1,2-dihydroxyéthyle, le 2,3-dihydroxypropyle, le 1,3-dihydroxypropyle, le 1,3-dihydroxybutyle, le 2,4-dihydroxybutyle, le 1,2,3-trihydroxypropyle, le 1,2,3-trihydroxybutyle. De préférence, R1 est choisi parmi l'hydroxyméthyle, le 1-hydroxyéthyle, le 2-hydroxyéthyle, le 1-méthyl-2-hydroxyéthyle, le 1,2-dihydroxyéthyle.

R2 est par exemple choisi parmi les groupes phényle, phényle substitué par des radicaux choisis parmi les radicaux halogène, alkyle en C1-C4, alcoxy en C1-C4, hydroxy, carboxyle, alkylthio en C1-C4, amino, alkylamino en C1-C4, dialkylamino en C1-C4, méthylènedioxy. De préférence, R2 est choisi parmi le phényle, le toluyle, le 2-, 3- ou 4-chlorophényle, le 3- ou 4-hydroxyphényle, le 3- ou 4-aminophényle, le 3- ou 4-méthoxyphényle. Selon un mode de réalisation particulièrement préféré, les groupes R2 sont choisis parmi le phényle, le toluyle, le 3- ou 4-hydoxyphényle, le 3- ou 4-aminophényle.

Dans les définitions ci dessus, les radicaux ou groupes alkyle sont linéaires ou ramifiés et comprennent, sauf autre indication, de 1 à 10 atomes de carbone, de préférence 1 à 6 atomes de carbone.

Parmi les pyrazolo[3,2-c]-1,2,4-triazoles de formule (I), on peut notamment citer les composés suivants ou leurs sels d'addition avec un acide ou une base:
le 6-hydroxyméthyl-3-phényl-pyrazolo[3,2-c]-1,2,4-triazole
le 6-hydroxyméthyl-3-(p-toluyl)-pyrazolo[3,2-c]-1,2,4-triazole
le 6-hydroxyméthyl-3-(o-toluyl)-pyrazolo[3,2-c]-1,2,4-triazole
le 6-hydroxyméthyl-3-(m-toluyl)-pyrazolo[3,2-c]-1,2,4-triazole
le 6-hydroxyméthyl-3-(3-hydroxyphényl)-pyrazolo[3,2-c]-1,2,4-triazole
le 6-hydroxyméthyl-3-(4-hydroxyphényl)-pyrazolo[3,2-c]-1,2,4-triazole
le 6-hydroxyméthyl-3-(3-aminophényl)-pyrazolo[3,2-c]-1,2,4-triazole
le 6-hydroxyméthyl-3-(4-aminophénylrpyrazolo[3,2-c]-1,2,4-triazole
le 6-(2-hydroxyéthyl)-3-phényl-pyrazolo[3,2-c]-1,2,4-triazole
le 6-(2-hydroxyéthyl)-3-(p-toluyl)-pyrazolo[3,2-c]-1,2,4-triazole
le 6-(2-hydroxyéthyl)-3-(o-toluyl)-pyrazolo[3,2-c]-1,2,4-triazole
le 6-(2-hydroxyéthyl)-3-(m-toluyl)-pyrazolo[3,2-c]-1,2,4-triazole
le 6-(2-hydroxyéthyl)-3-(3-hydroxyphényl)-pyrazolo[3,2-c]-1,2,4-triazole
le 6-(2-hydroxyéthyl)-3-(4-hydroxyphényl)-pyrazolo[3,2-c]-1,2,4-triazole
le 6-(2-hydroxyéthyl)-3-(3-aminophényl)-pyrazolo[3,2-c]-1,2,4-triazole
le 6-(2-hydroxyéthyl)-3-(4-aminophényl)-pyrazolo[3,2-c]-1,2,4-triazole
le 6-(1-hydroxyéthylr3-phényl-pyrazolo[3,2-c]-1,2,4-triazole
le 6-(1-hydroxyéthyl)-3-(p-toluyl)-pyrazolo[3,2-c]-1,2,4-triazole
le 6-(1-hydroxyéthyl)-3-(o-toluyl)-pyrazolo[3,2-c]-1,2,4-triazole
le 6-(1-hydroxyéthyl)-3-(m-toluyl)-pyrazolo[3,2-c]-1,2,4-triazole
le 6-(1-hydroxyéthyl)-3-(3-hydroxyphényl)-pyrazolo[3,2-c]-1,2,4-triazole
le 6-(1-hydroxyéthyl)-3-(4-hydroxyphényl)-pyrazolo[3,2-c]-1,2,4-triazole
le 6-(1-hydroxyéthyl)-3-(3-aminophényl)-pyrazolo[3,2-c]-1,2,4-triazole
le 6-(1-hydroxyéthyl)-3-(4-aminophényl)-pyrazolo[3,2-c]-1,2,4-triazole

De préférence, les pyrazolo[3,2-c]-1,2,4-triazoles de formule (I) sont choisis parmi les composés suivants ou leurs sels d'addition avec un acide ou une base:
le 6-hydroxyméthyl-3-phényl-pyrazolo[3,2-c]-1,2,4-triazole
le 6-hydroxyméthyl-3-(p-toluyl)-pyrazolo[3,2-c]-1,2,4-triazole
le 6-hydroxyméthyl-3-(o-toluyl)-pyrazolo[3,2-c]-1,2,4-triazole
le 6-hydroxyméthyl-3-(m-toluyl)-pyrazolo[3,2-c]-1,2,4-triazole
le 6-hydroxyméthyl-3-(3-hydroxyphényl)-pyrazolo[3,2-c]-1,2,4-triazole
le 6-hydroxyméthyl-3-(4-hydroxyphényl)-pyrazolo[3,2-c]-1,2,4-triazole
le 6-hydroxyméthyl-3-(3-aminophényl)-pyrazolo[3,2-c]-1,2,4-triazole
le 6-hydroxyméthyl-3-(4-aminophényl)-pyrazolo[3,2-c]-1,2,4-triazole

Les composés de la présente invention peuvent être obtenus à partir des procédés de préparation décrits par exemple dans la demande de brevet FR 2 746 306, ainsi que dans les publications suivantes : Chem. Ber. 32, 797 (1899), Chem. Ber. 89, 2550, (1956), J. Chem. Soc. Perkin trans I, 2047, (1977), J. Prakt. Chem., 320, 533, (1978), J. fur Chem., 326(5), 829, (1984).

L'invention a aussi pour objet une composition pour la teinture des fibres kératiniques et en particulier des fibres kératiniques humaines telles que les cheveux, comprenant, dans un milieu approprié pour la teinture, au moins un coupleur pyrazolotriazole de formule (1) ou l'un de ces sels d'addition avec un acide ou une base tel que défini précédemment et au moins une base d'oxydation.

La base d'oxydation est choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques, et leurs sels d'addition avec un acide.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paràphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β(3-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylaminoréthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Dans la composition de la présente invention, le ou les coupleurs pyrazolotriazoles sont présents en quantité de préférence comprise entre 0,001 et 10 % en poids environ du poids total de la composition tinctoriale et encore plus préférentiellement de 0,005 à 6 %, et la ou les bases d'oxydation sont présentes en quantité de préférence comprise entre 0,001 à 10 % en poids environ du poids total de la composition tinctoriale, et encore plus préférentiellement de 0,005 à 6 %.

La composition selon l'invention peut contenir en plus du coupleur pyrazolo-triazole de formule (I), un ou plusieurs coupleurs additionnels qui sont conventionnellement utilisés pour la teinture de fibres kératiniques. Parmi ces coupleurs, on peut notamment citer les métaphénylènediamines, les métaaminophénols, les métadiphénols et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsqu'ils sont présents, le ou les coupleurs additionnels représentent de préférence de 0,001 à 10 % en poids environ du poids total de la composition tinctoriale et encore plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates. Les sels d'addition avec une base utilisables dans le cadre de l'invention sont par exemple choisi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également comprendre divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, associatifs ou non des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 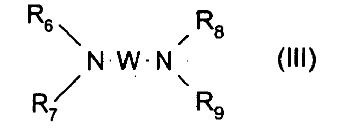 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition selon la présente invention telle que définie précédemment, la couleur étant révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale définie ci-dessus et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a enfin pour objet un procédé de synthèse d'un composé de formule (II) à partir d'un composé de formule (III) 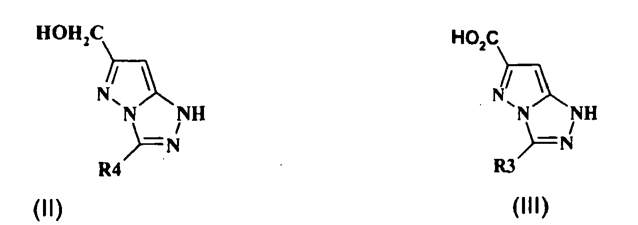 formules dans lesquelles R3 et R4, identiques ou différents, représentent un groupe aryle, par exemple phényle ou naphtyle, pouvant être substitué par au moins un radical choisi dans le groupe constitué par les radicaux halogène, alkyle, hydroxyle, alcoxy, amino, alkylamino, dialkylamino et alkylthio, le procédé comprenant une étape de réduction de la fonction carboxylique en fonction alcool.

R3 et R4 sont par exemple choisis parmi les groupes phényle, phényle substitué par un ou deux groupes choisis parmi les radicaux halogène, alkyle en C1-C2, alcoxy en C1-C2, hydroxy, alkylthio en C1-C2, amino, alkylamino en C1-C2, dialkylamino en C1-C2, méthylènedioxy. De préférence, R3 et R4 sont choisis parmi le phényle, le toluyle, le 2-, 3- ou 4-chlorophényle, le 3- ou 4-hydroxyphényle, le 3- ou 4-aminophényle, le 3- ou 4-méthoxyphényle. Selon un mode de réalisation particulièrement préféré, les groupes R3 et R4 sont choisis parmi le phényle, le toluyle, le 3- ou 4-hydoxyphényle, le 3- ou 4-aminophényle.

Le procédé de synthèse est mis en oeuvre en une ou plusieurs étapes en fonction de la nature du substituant R3.

Lorsque les groupes R3 et R4 sont différents, le procédé comprend une ou plusieurs étapes supplémentaires visant à transformer R3 en R4. De plus, le composé (III) peut être transformé en ester avant l'étape de réduction. Une telle modification peut être utile pour augmenter la solubilité du composé (III) et faciliter l'a réduction.

Ainsi, lorsque R3 et R4 sont identiques, la réduction de la fonction acide carboxylique en position 6 en fonction alcool peut être réalisée de façon directe en une étape par les réducteurs usuels tels que les hydrures métalliques, par exemple LiAlH₄, le DIBAL, NaAlEt₂H₂, LiAlH(OMe)₃ ou le borane, ces réactifs étant introduits au moins en quantité stoechiométrique. Les solvants utilisés sont par exemple le THF, les éthers ou le DME. Cette voie de synthèse est représentée par le schéma 1 ci-dessous.

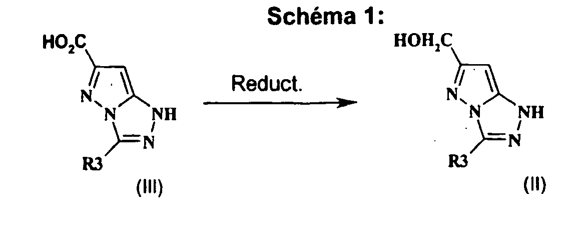

Selon la nature du substituant R3, la solubilité du composé (III) peut être faible dans les solvants dipolaires aprotiques. Il peut être avantageux dans ce cas de transformer dans une première étape la fonction acide carboxylique du composé (III) en une fonction ester méthylique ou éthylique pour augmenter la solubilité du composé (III), puis dans une seconde étape, de réduire la fonction ester par les réducteurs classiques dans un solvant dipolaire aprotique pour obtenir le composé (II) correspondant. Ce mode de réalisation est représenté sur le schéma 2 ci dessous. La préparation de l'ester méthylique, respectivement éthylique, est réalisée par les méthodes usuelles comme par exemple par réaction de la fonction acide carboxylique dans le méthanol, respectivement dans l'éthanol, en milieu acide.

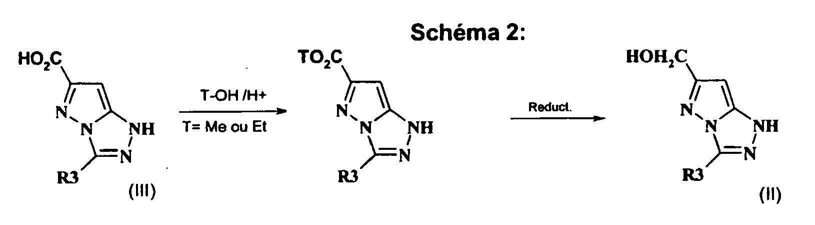

Lorsque R3 et R4 sont différents, les modifications chimiques du substituant R3 peuvent être réalisées soit sur le composé (II) soit sur le composé (III) selon la nature des modifications à apporter ou selon leur facilité de réalisation sur l'un ou l'autre des composés. Dans ce cas, la préparation du composé (II) à partir du composé (III) peut être réalisée par l'un ou l'autre des schémas 3 à 6 ci dessous

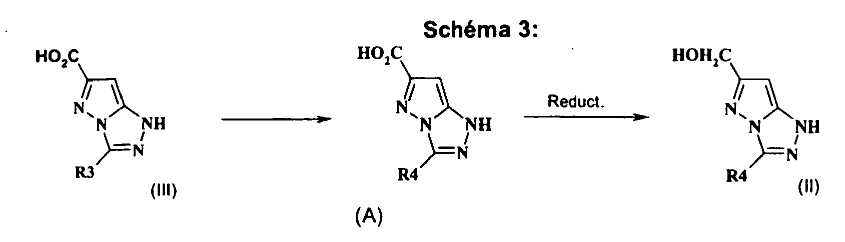

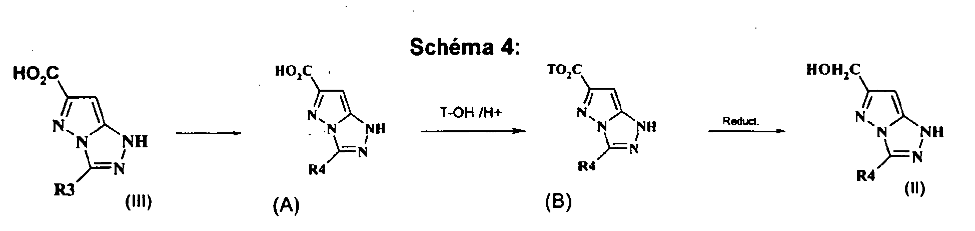

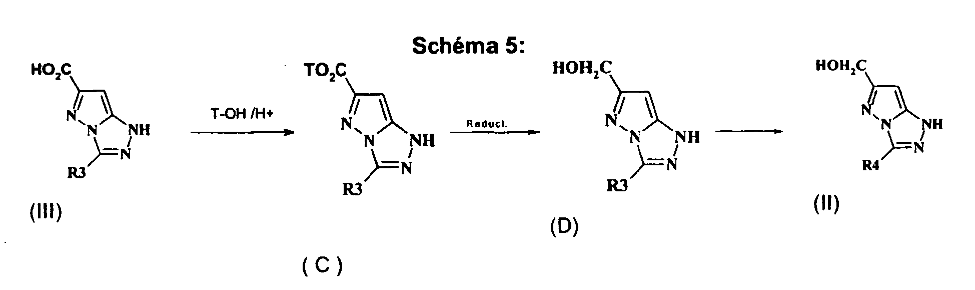

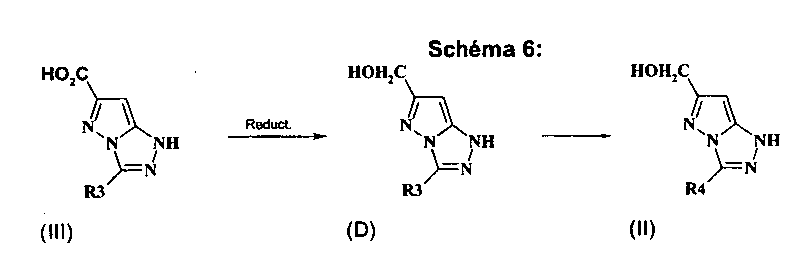

Selon le schéma 3, le composé (III) est transformé en composé A dans les conditions opératoires nécessaires pour transformer R3 en R4. Le composé A est ensuite réduit dans les conditions opératoires déjà décrites pour obtenir le composé (II).

Selon le schéma 4, la fonction acide carboxylique du composé (III) est transformée en ester méthylique ou éthylique pour former le composé B selon les méthodes précédemment décrites. Le composé B est alors réduit dans les conditions opératoires déjà décrites pour obtenir le composé (II).

Un mode de réalisation particulier est décrit sur le schéma 5, selon lequel on prépare dans une première étape l'ester méthylique ou éthylique du composé (II) par une des méthodes déjà citées pour synthétiser le composé C plus soluble que le composé (II) dans les solvants dipolaires aprotiques. Le composé C est alors réduit par exemple au moyen d'un hydrure métallique ou le borane comme déjà indiqué, pour aboutir au composé D. On peut ensuite réaliser la modification chimique souhaitée pour transformer le substituant R3 en substituant R4. Si la solubilité du composé (III) est suffisante, on peut également directement réduire la fonction acide carboxylique en position 6 pour préparer le composé D selon une des méthodes déjà citées. Le composé (II) est alors préparé à partir du composé D. Cette variante est représentée sur le schéma 6.

Selon un mode de réalisation particulier, la synthèse du 6-hydroxyméthyl-3-phényl-pyrazolo[3,2-c]-1,2,4-triazole de formule (II) peut être réalisée directement à partir du 6-carboxy-3-phényl-pyrazolo[3,2-c]-1,2,4-triazole (III). II est de préférence obtenu selon le mode opératoire du schéma 2 qui consiste à transformer préalablement le 6-carboxy-3-phényl-pyrazolo[3,2-c]-1,2,4-triazole en 6- carboxyméthyl-3-phénylpyrazolo[3,2-c]-1,2,4-triazole. Le 6-hydroxyméthyl-3-phényl-pyrazolo[3,2-c]-1,2,4-triazole peut également être préparé à partir du 6-carboxy-3-(3',4'-dichlororphénylpyrazolo[3,2-c]-1,2,4-triazole selon un des schémas 3,4,5 ou 6.

Selon le schéma 3, le 6-carboxy-3-(3',4'-dichloro)-phényl-pyrazolo[3,2-c]-1,2,4-triazole est transformé en 6-carboxy-3-phényl-pyrazolo[3,2-c]-1,2,4-triazole par exemple par réduction catalytique en présence d'un catalyseur métallique tel que le palladium ou l'hydroxyde de palladium sous une pression d'hydrogène de 2 à 100 bars, préférentiellement de 2 à 60 bars, à une température variant de 10 à 90°C, préférentiellement entre 20 et 60°C. La réaction est préférentiellement réalisée en milieu basique contenant par exemple une base organique telle qu'une amine primaire, secondaire ou tertiaire, par exemple la triéthylamine, féthyldüsopropylamine ou la butylamine, ou une base minérale telle que l'hydroxyde de sodium, l'hydroxyde de potassium, le carbonate de potassium, le carbonate de sodium, l'hydrogénocarbonate de potassium, l'hydrogénocarbonate de sodium, ou un mélange de ces bases. L'hydrogénation est réalisée dans un solvant tel qu'un alcool comme l'éthanol ou le méthanol, dans l'eau, dans un mélange hydroalcoolique ou dans un mélange d'eau et de solvant dipolaire aprotique tel que le dioxane ou le THF. Préférentiellement, la réaction est réalisée dans un mélange eau - solvant dipolaire aprotique. Le 6-carboxy-3-phényl-pyrazolo[3,2-c]-1,2,4-triazole est ensuite réduit directement en 6-hydroxyméthyl-3-phényl-pyrazolo[3,2-c]-1,2,4-triazole (schéma 3) ou d'abord estérifié en ester méthylique ou éthylique avant d'être réduit (schéma 4).

Selon le schéma 5, le 6-carboxy-3-(3',4'-dichloro)-phényl-pyrazolo[3,2-c]-1,2,4-triazole est tout d'abord estérifié en composé C, le composé C est ensuite réduit pour former le 6-hydroxyméthyl-3-(3',4'-dichloro)-phényl-pyrazolo[3,2-c]-1,2,4-triazole. La transformation du substituant R3 =1-(3',4'-dichloro)-phényl en R4 = phényl est réalisée par hydrogénation catalytique dans les conditions déjà décrites.

Selon le schéma 6, le carboxy en position 6 est d'abord réduit en hydroxyméthyle puis le 6- hydroxyméthyl-3-(3',4'-dichloro)-phényl-pyrazolo[3.2-c]-1,2.4-triazole est transformé en 6- hydroxyméthyl-3-phényl-pyrazolo[3,2-c]-1,2,4-triazole par réduction catalytique dans les conditions précitées.

L'invention a enfin pour objet un produit coloré susceptible d'être obtenu par réaction d'au moins un coupleur pyrazolo--[3,2-c]-1,2,4-triazole de formule (1) ou l'un de ses sels d'addition avec un acide ou une base tel que défini précédemment, et au moins une base d'oxydation

Les exemples qui suivent servent à illustrer l'invention sans toutefois présenter un caractère limitatif.

### EXEMPLES

### EXEMPLE 1: Synthèse du 6-hydroxyméthyl-3-phényl - pyrazolo [3,2-c]-1,2,4-triazole (H) selon le schéma 3

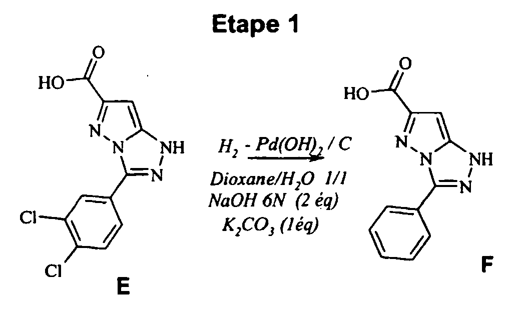

On dissout 60g de 6-carboxy-3-(3',4'-dichloro)-phényl-pyrazolo[3,2-c]-1,2,4-triazole **E** et 28,05 g de carbonate de potassium dans 1,8 litres de mélange composé de 69ml de soude 6N et de dioxane à 50% dans l'eau. Cette solution est transférée dans un hydrogénateur et additionnée de 9g de Pd(OH)₂/C à 20% et 50% d'humidité. Le milieu est purgé deux fois par l'azote avant introduction de 35 bars d'hydrogène, puis porté à 35°C pendant 8 heures. Après élimination de l'hydrogène, le milieu est filtré. Le filtrat est partiellement concentré pour éliminer le dioxane et la solution aqueuse résiduelle est acidifiée par une solution aqueuse d'acide chlorhydrique dilué jusqu'à pH égal à 2. Le précipité ainsi obtenu est filtré, essoré, lavé par de l'eau distillée et séché sous vide. On obtient ainsi 45g de composé F sous forme de cristaux blancs (rendement 97,6%).
Analyse élémentaire:

| | C | H | N | O |
|---|---|---|---|---|
| Calculé | 57,90 | 3,53 | 24,55 | 14,02 |
| Trouvé | 57,30 | 3,47 | 24,35 | 14,38 |

RMN (1H, 400MHz, DMSO d6): 6,38 (s,1 H), 7,56(m, 1H), 7,62 (dd, 2H), 8,43 (dd, 2H), 12,98 (se, 1H, 13,45 (s, 1 H).
RMN (13C, 39.5MHz, DMSO d6): 80,65, 125,37, 125,59, 128,99, 130,10, 137,59, 147,56, 151,05, 163,75.

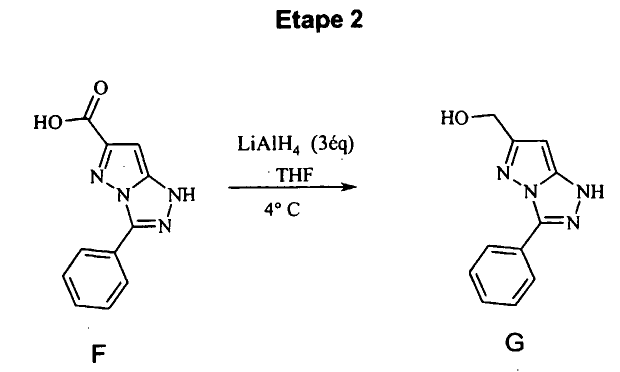

Sous gaz inerte, on solubilise à température ambiante 1g de composé F dans 400ml de THF anhydre. Après refroidissement à 0°C, on ajoute par petites fractions 333 mg de LiAlH₄ en 1 heure. Le milieu réactionnel s'opacifie et blanchit. On laisse remonter la température à température ambiante. La réaction est suivie par CCM.

Après 3h30, on ajoute à nouveau 0,2 éq LiAlH₄ (33mg) à température ambiante puis 1 heure après à nouveau 0,4 éq de LiAlH₄ (66mg) et enfin après 45mn, on procède à un nouvel ajout de 0,4 éq (66 mg) de LiAlH₄ et on laisse sous agitation à température ambiante. Après une nuit, la réaction est terminée.

On acidifie jusqu'à pH égal à 3 par HCl,1M le milieu réactionnel qui se décolore progressivement. Le milieu est ensuite extrait par l'acétate d'éthyle. Un précipité grisâtre apparaît, il est filtré sur verre fritté n° 4. L'extraction est poursuivie à l'aide d'ajout de solution aqueuse saturée de NaCl. La phase organique est séchée sur sulfate de sodium, filtrée et concentrée à sec. Un solide orange est ainsi obtenu. Ce dernier est lavé par Et₂O puis AcOEt et filtré sur verre fritté n° 4. On obtient ainsi une poudre beige-rosé après séchage sous vide. On isole ainsi 360 mg de composé G avec un rendement de 38,4%.
Analyse élémentaire:

| | C | H | N | O |
|---|---|---|---|---|
| Calculé | 61.67 | 4.71 | 26.15 | 7.47 |
| Trouvé | 60.31 | 4.69 | 25.90 | 8.45 |

### EXEMPLE 2 : Synthèse du 6-hydroxyméthyl-3-phényl - pyrazolo [3,2-c]-1,2,4-triazole (H) selon le schéma 4

### Etape 1

On reproduit l'étape 1 de l'exemple 1 pour obtenir le composé F.

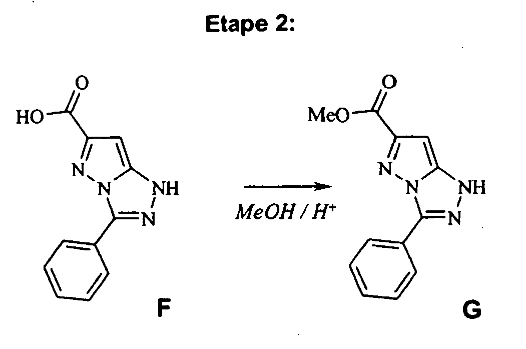

Sous atmosphère inerte, on dissout 30 g de composé F à température ambiante dans 6 litres de méthanol anhydre. On ajoute 3g d'acide 10-camphosulfonique puis on chauffe au reflux pendant 48 heures. On laisse revenir à température ambiante, le composé G précipite au refroidissement. Le composé G ainsi formé est filtré, essoré et lavé plusieurs fois par de l'éther éthylique avant d'être séché sous vide. On obtient ainsi 23,88g d'ester méthylique G sous forme de poudre avec un rendement de 75%

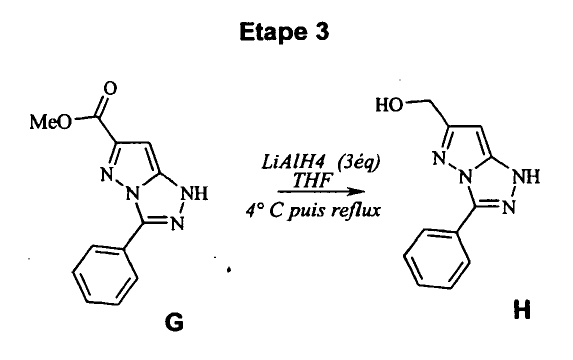

Sous atmosphère inerte, on introduit 2,33 litres de THF anhydre puis 20 grammes d'ester G. Après 15 min d'agitation, le milieu est refroidi à 0°c et 10,6 g de LiAIH₄ sont additionnés par petites fractions en 30 min en maintenant la température à 0°C. Le milieu est ensuite porté au reflux pendant 4h30 puis abandonné 1 nuit à température ambiante. Le milieu est alors à nouveau refroidi à 0°C et hydrolysé. Le précipité obtenu est filtré et essoré. Le filtrat est concentré sous vide jusqu'à un volume de 200 ml avant d'être acidifié jusqu'à pH égal à 3 par une solution aqueuse d'acide chlorhydrique dilué. Le précipité de composé H ainsi formé est filtré, essoré et séché sous vide conduisant à 15,5g de composé H sous forme de poudre beige avec un rendement de 87,6%.

### EXEMPLES DE TEINTURE

### Exemples 3 à 5

On a préparé les compositions tinctoriales suivantes (teneurs en moles) :

| **Exemples** | **3** (Inv) | **4** (Inv) | **5** (Inv) |
|---|---|---|---|
| 6-hydroxyméthyl-3-phényl - pyrazolo [3,2-c]-1,2,4-triazole | 3x10⁻³ | 3x10⁻³ | 3x10⁻³ |
| Paraphénylène diamine | 3x10⁻³ | - | - |
| 3,7 diaminopyrazolo[1,5-a]pyrimidine, 2HCl | - | 3x10⁻³ | |
| N, N-bis-(β-(3-hydroxyéthyl) paraphénylènediamine, H₂SO₄, H₂O | - | - | 3x10⁻³ |
| Support de teinture (1) | (*) | (*) | (*) |
| Eau déminéralisée q.s.p. | 100 g | 100 g | 100 g |

### Support de teinture (1)

| | |
|---|---|
| alcool éthylique à 96° | 18 g |
| Métabisulfite de sodium en solution aqueuse à 35% | 0,68 g |
| sel pentasodique de l'acide diéthylène triamino pentacétique | 1,1g |
| ammoniaque à 20% | 10g |

Au moment de l'emploi, chaque composition des exemples 3 à 5 est mélangée avec un poids égal d'eau oxygénée à 20 volumes (6% en poids).

Chaque mélange obtenu est appliqué sur des mèches de cheveux gris à 90 % de blancs, permanentés (BP) ou naturel (BN). Après 30 min de pose, les mèches sont rinçées, lavées avec un shampooing standard, rinçées à nouveau puis séchées.

Les résultats de teinture suivants ont été obtenus.

| | **BN** | **BP** |
|---|---|---|
| Exemple 3 | Violine lgt cendré | Violine lgt cendré |
| Exemple 4 | Irisé rouge lgt cuivré | Rouge irisé |
| Exemple 5 | Violet | Violet intense |

### Exemples 6 et 7

On a préparé les compositions tinctoriales suivantes (teneurs en moles) :

| **Exemples** | **6 (inv)** | **7 (comp)** |
|---|---|---|
| 6-hydroxyméthyl-3-phényl - pyrazolo [3,2-c]-1,2,4-triazole | 6,4×10⁻³ | |
| N,N-bis-(β-hydroxyéthyl) paraphénylènediamine, H₂SO₄, H₂O | 6,4×10⁻³ | 6,4×10⁻³ |
| 4,6-Dimethyl-2H-pyrazolo[3,2c]-1,2,4-triazole | | 6,4×10⁻³ |
| Support de teinture (2) | (*) | (*) |
| Eau déminéralisée q.s.p. | 100 g | 100 g |

### Support de teinture (2)

- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 par la société AKZO 7,0g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,0 g

Au moment de l'emploi, chaque composition des exemples 6 et 7 est mélangée avec un poids égal d'eau oxygénée à 20 volumes (6% en poids).

Chaque mélange obtenu est appliqué sur des mèches de cheveux gris à 90 % de blancs, permanentés (BP) ou naturels (BN). Après 30 min de pose, lés mèches sont rinçées, lavées avec un shampooing standard, rinçées à nouveau puis séchées.

La couleur des mèches est évaluée au moyen d'un spectrophotomètre CM 2002 MINOLTA. Les nuances mesurées au MINOLTA CM 2002 sont chiffrées en valeurs MUNSELL (Norme ASTM D 1535-68), dans lesquelles la valeur H désigne la nuance ou HUE, la valeur V désigne l'intensité ou VALUE, et la valeur C la pureté ou CHROMATICITE.

Les résultats sont reportés dans le tableau ci dessous.

| **Exemples** | **Cheveux blancs naturels** | | | **Cheveux blancs permanentés** | | |
|---|---|---|---|---|---|---|
| | **H** | **V** | **C** | **H** | **V** | **C** |
| Exemple 6 | 3,1P | 3 | 4,2 | 3P | 2,2 | 4,8 |
| Exemple 7 | 0,4R | 4,1 | 2 | 6,4RP | 3,4 | 2,7 |

Ces résultats montrent que le coupleur 6-hydroxyméthyl-3-phényl - pyrazolo [3,2-c]-1,2,4-triazole permet d'obtenir des reflets violets plus chromatiques.

### Exemples 8 et 9 : Application différée

On a préparé les compositions suivantes (moles)

| **Exemples** | **8** | **9** |
|---|---|---|
| 6-hydroxyméthyl-3-phényl - pyrazolo [3,2-c]-1,2,4-triazole | 3x10⁻³ | - |
| 4,6-Dimethyl-2H-pyrazolo[3,2c]-1,2,4-triazole (Comp) | - | 3×10⁻³ |
| N,N-bis-(β-hydroxyéthyl) paraphénylènediamine H₂SO₄, H₂O | 3x10⁻³ | 3x10⁻³ |
| Support de teinture (3) | (*) | (*) |
| Eau déminéralisée q.s.p. | 100 g | 100 g |

### Support de teinture (3)

| | |
|---|---|
| alcool éthylique à 96° | 18 g |
| Métabisulfite de sodium en solution aqueuse à 35% | 0,68 g |
| sel pentasodique de l'acide diéthylène triamino pentacétique | 1,1g |
| ammoniaque à 20% | 10g |

Les compositions des exemples 8 à 9 sont mélangées avec un poids égal d'eau oxygénée à 20 volumes (6% en poids). Une partie de chacune des compositions est appliquée sur des mèches de cheveux gris à 90 % de cheveux blancs naturels (BN) ou permanentés (BP) au moment du mélange (T0), une seconde partie est appliquée sur de nouvelles mèches 15 min après le mélange (T15) et une troisième partie est appliquée 30 minutes après le mélange (T30). L'application sur les mèches est effectuée selon le mode opératoire décrit précédemment.

Les résultats suivants ont été obtenus.

| | | | | |
|---|---|---|---|---|
| | | **PUISSANCE DE COUPLAGE** | **90% BN Reflets** | **90% BP Reflets** |
| Exemple 9 | T0 | 4 | Irisé violet intense | Violet Igt irisé intense |
| | T15 | 3,5 | Irisé violet | Violet Igt irisé intense |
| | T30 | 3 | Irisé lgt violet lgt cendré | Violet lgt irisé |
| Exemple 8 | T0 | 4 | Violet bleuté très lgt irisé | Violet bleuté intense |
| | T15 | 4 | Violet bleuté très lgt irisé cendré | Violet bleuté très Igt cendré |
| | T30 | 4 | Violet bleuté très Igt cendré irisé | Violet bleuté très Igt cendré |

La puissance de couplage correspond à la classification des nuances naturelles (voir "Science des Traitements Capillaires" de C. ZVIAK, Ed. Masson 1988, p. 278). Une puissance de 3 correspond à un châtain foncé, une puissance de 4 correspond à un châtain.

Ces résultats montrent que le coupleur 6-hydroxyméthyl-3-phényl - pyrazolo [3,2-c]-1,2,4-triazole présente en application différée une variation moindre de la coloration.

## Revendications

1. Pyrazolo-[3,2-c]-1,2,4-triazole de formule (1) ou l'un de ses sels d'addition avec un acide ou une base: 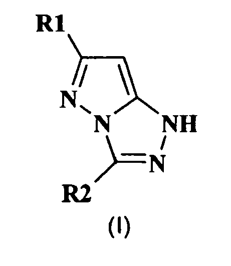 dans laquelle
R1 représente un groupe hydroxy ou un groupe alkyle en C1-C4, substitué par au moins un radical hydroxy, et
R2 représente un groupe aryle pouvant être substitué par au moins un radical choisi parmi le groupe constitué par les radicaux halogène, nitro, cyano, alkyle, hydroxyle, alcoxy, amino, alkylamino, dialkylamino, acylamino, sulfonamido, alkylthio, alcoxycarbonyle, carboxy et sulfonique.

2. Pyrazolo-[3,2-c]-1,2,4-triazole selon la revendication 1 dans lequel R1 est un groupe alkyle en C1-C4, substitué par 1 à 3 radicaux hydroxyle et R2 est un groupe phényle pouvant être substitué par 1 à 3 radicaux.

3. Pyrazolo-[3,2-c]-1,2,4-triazole selon la revendication 2 dans lequel R1 est choisi parmi l'hydroxyméthyle, le 2-hydroxyéthyle, le 3-hydroxypropyle, le 1-hydroxyéthyle, le 1-hydroxypropyle, le 1-méthyl-2-hydroxyéthyle, le 1,2-dihydroxyéthyle, le 2,3-dihydroxypropyle, le 1,3-dihydroxypropyle, le 1,3-dihydroxybutyle, le 2,4-dihydroxybutyle, le 1,2,3-trihydroxypropyle, le 1,2,3-trihydroxybutyle.

4. Pyrazolo-[3,2-c]-1,2,4-triazole selon la revendication 2 dans lequel R2 est choisi parmi les groupes phényle, phényle substitué par un ou deux radicaux choisis parmi les radicaux halogène, alkyle en C1-C4, alcoxy en C1-C4, hydroxy, carboxyle, alkylthio en C1-C4, amino, alkylamino en C1-C4, dialkylamino en C1-C4, méthylènedioxy.

5. Pyrazolo-[3,2-c]-1,2,4-triazole selon l'une quelconque des revendications précédentes, dans lequel les sels d'addition avec un acide sont choisis parmi les chlorhydrates, les bromhydrates, les sulfates, les tartrates, les benzènesulfonates, les lactates, les tosylates et les acétates, et les sels d'addition avec une base sont choisie parmi les sels d'addition avec la soude, la potasse, l'ammoniaque, les amines ou les alcanolamines.

6. Pyrazolo-[3,2-c]-1,2,4-triazole selon l'une quelconque des revendications précédentes choisi parmi les composés suivants ou leurs sels d'addition avec un acide ou une base :
le 6-hydroxyméthyl-3-phényl-pyrazolo[3,2-c]-1,2,4-triazole
le 6-hydroxyméthyl-3-(p-toluyl)-pyrazolo[3,2-c]-1,2,4-triazole
le 6-hydroxyméthyl-3-(o-toluyl)-pyrazolo[3,2-c]-1,2,4-triazole
le 6-hydroxyméthyl-3-(m-toluyl)-pyrazolo[3,2-c]-1,2,4-triazole
le 6-hydroxyméthyl-3-(3-hydroxyphényl)-pyrazolo[3,2-c]-1,2,4-triazole
le 6-hydroxyméthyl-3-(4-hydroxyphénylrpyrazolo[3,2-c]-1,2,4-triazole
le 6-hydroxyméthyl-3-(3-aminophényl)-pyrazolo[3,2-c]-1,2,4-triazole
le 6-hydroxyméthyl-3-(4-aminophényl)-pyrazolo[3,2-c]-1 ,2,4-triazole
le 6-(2-hydroxyéthyl)-3-phényl-pyrazolo[3,2-c]-1,2,4-triazole
le 6-(2-hydroxyéthyl)-3-(p-toluyl)-pyrazolo[3,2-c]-1,2,4-triazole
le 6-(2-hydroxyéthyIY3-(o-toluyl)-pyrazolo[3,2-c]-1,2, 4-triazole
le 6-(2-hydroxyéthyl)-3-(m-toluyl)-pyrazolo[3,2-c]-1,2,4-triazole
le 6-(2-hydroxyéthyl)-3-(3-hydroxyphényl)-pyrazolo[3,2-c]-1,2,4-triazole
le 6-(2-hydroxyéthyl)-3-(4-hydroxyphényl)-pyrazolo[3,2-c]-1,2,4-triazole
le 6-(2-hydroxyéthyl)-3-(3-aminophényf)-pyrazolo[3,2-c]-1,2,4-triazole
le 6-(2-hydroxyéthyl)-3-(4-aminophényl)-pyrazolo[3,2-c]-1,2,4-triazole
le 6-(1-hydroxyéthyl)-3-phényl-pyrazolo[3,2-c]-1,2,4-triazole
le 6-(1-hydroxyéthyl)-3-(p-toluyl)-pyrazolo[3,2-c]-1,2,4-triazole
le 6-(1-hydroxyéthyl)-3-(o-toluyl)-pyrazolo[3,2-c]-1,2,4-triazole
le 6-(1-hydroxyéthyl)-3-(m-toluyl)-pyrazolo[3,2-c]-1,2,4-triazole
le 6-(1-hydroxyéthyl)-3-(3-hydroxyphényl)-pyrazolo[3,2-c]-1,2,4-triazole
le 6-(1-hydroxyéthyl)-3-(4-hydroxyphényl)-pyrazolo[3,2-c]-1,2,4-triazole
le 6-(1-hydroxyéthyl)-3-(3-aminophénylrpyrazolo[3,2-c]-1,2,4-triazole
le 6-(1-hydroxyéthyl)-3-(4-aminophényl)-pyrazolo[3,2-c]-1,2,4-triazole.

7. Pyrazolo-[3,2-c]-1,2,4-triazole selon la revendication 6 choisi parmi les composés suivants ou leurs sels d'addition avec un acide ou une base :
le 6-hydroxyméthyl-3-phényl-pyrazolo[3,2-c]-1,2,4-triazole
le 6-hydroxyméthyl-3-(p-toluyl)-pyrazolo[3,2-c]-1,2,4-triazole
le 6-hydroxyméthyl-3-(o-toluyl)-pyrazolo[3,2-c]-1,2,4-triazole
le 6-hydroxyméthyl-3-(m-toluyl)-pyrazolo[3,2-c]-1,2,4-triazole.
le 6-hydroxyméthyl-3-(3-hydroxyphényl)-pyrazolo[3,2-c]-1,2,4-triazole
le 6-hydroxyméthyl-3-(4-hydroxyphényl)-pyrazolo[3,2-c]-1,2,4-triazole
le 6-hydroxyméthyl-3-(3-aminophényl)-pyrazolo[3,2-c]-1,2,4-triazole
le 6-hydroxyméthyl-3-(4-aminophényl)-pyrazolo[3,2-c]-1,2,4-triazole.

8. Composition pour la teinture des fibres kératiniques et en particulier des fibres kératiniques humaines telles que les cheveux, comprenant, dans un milieu approprié pour la teinture, au moins un coupleur pyrazolo-[3,2-c]-1,2,4-triazole de formule (I) ou l'un de ses sels d'addition avec un acide ou une base tel que défini selon l'une quelconque des revendications 1 à 7 et au moins une base d'oxydation.

9. Composition selon la revendication 8 dans laquelle le coupleur pyrazolo-triazole est tel que R1 est un groupe alkyle en C1-C4, substitué par 1 à 3 radicaux hydroxyle et R2 est un groupe phényle pouvant être substitué par 1 à 3 radicaux.

10. Composition selon l'une quelconque des revendications 8 ou 9, dans laquelle le ou les coupleurs pyrazolo-t-[3,2-c]-1,2,4-triazoles de formule (I) sont présents en quantité comprise entre 0,001 et 10 %, de préférence entre 0,005 et 6 %.

11. Composition selon l'une quelconque des revendications 8 à 10, dans laquelle la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

12. Composition selon l'une quelconque des revendications 8 à 11, dans laquelle la ou les bases d'oxydation sont présentes en quantité comprise entre 0,001 et 10 %, de préférence entre 0,005 et 6 %.

13. Composition selon l'une quelconque des revendications 8 à 12 comprenant de plus un colorant direct.

14. Procédé de teinture d'oxydation des fibres kératiniques et en particulier des fibres kératiniques humaines telles que les cheveux, **caractérisé en ce qu'**on applique sur les fibres au moins une composition telle que définie à l'une quelconque des revendications 8 à 13, et qu'on révèle la couleur à l'aide d'un agent oxydant.

15. Procédé selon la revendication 14 dans lequel l'agent oxydant est choisi parmi le peroxyde d'hydrogène, le peroxyde d'urée, les bromates de métaux alcalins, les persels tels que les perborates et persulfates, les peracides et les enzymes oxydases.

16. Procédé selon l'une des revendications 14 ou 15 dans lequel l'agent oxydant est mélangé au moment de l'emploi à la composition telle que définie selon l'une quelconque des revendications 8 à 13.

17. Procédé selon l'une quelconque des revendications 14 ou 15 dans lequel l'agent oxydant est appliqué sous forme de composition oxydante simultanément ou séquentiellement à la composition telle que définie selon l'une quelconque des revendications 6 à 13 sur les fibres.

18. Dispositif à plusieurs compartiments ou "kit" de teinture à plusieurs compartiments, dans lequel un premier compartiment contient une composition telle que définie à l'une quelconque des revendications 8 à 13 et un deuxième compartiment contient une composition oxydante.

19. Produit coloré susceptible d'être obtenu par réaction d'au moins un coupleur pyrazolo-[3,2-c]-1,2,4-triazole de formule (I) ou l'un de ses sels d'addition avec un acide ou une base tel que défini selon l'une quelconque des revendications 1 à 7, et au moins une base d'oxydation.

20. Procédé de préparation d'un composé de formule (II) 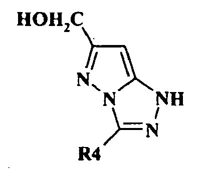 à partir d'un composé de formule (III) 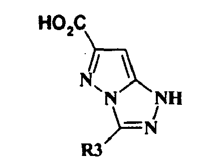 formules dans lesquelles R3 et R4, identiques ou différents, représentent un groupe aryle pouvant être substitué par au moins un radical choisi dans le groupe constitué par les radicaux halogène, alkyle, hydroxyle, alcoxy, amino, alkylamino, dialkylamino et alkylthio, le procédé comprenant une étape de réduction de la fonction carboxylique du composé (III) en fonction alcool.

21. Procédé selon la revendication 20 comprenant une ou plusieurs étapes intermédiaires visant à transformer R3 en R4, lorsque R3 est différent de R4.

22. Procédé selon la revendication 20 ou 21 dans lequel la fonction acide carboxylique du composé (III) est transformée en fonction ester avant l'étape de réduction.

## Claims

1. Pyrazolo[3,2-c]-1,2,4-triazole of formula (I) or one of its addition salts with an acid or a base: 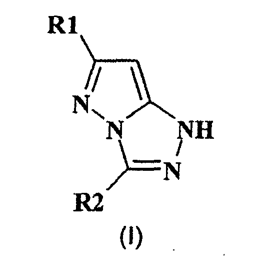 in which
R1 represents a hydroxyl group or a C1-C4 alkyl group substituted with at least one hydroxyl radical, and
R2 represents an aryl group which may be substituted with at least one radical chosen from the group consisting of halogen, nitro, cyano, alkyl, hydroxyl, alkoxy, amino, alkylamino, dialkylamino, acylamino, sulphonamido, alkylthio, alkoxycarbonyl, carboxy and sulpho radicals.

2. Pyrazolo[3,2-c]-1,2,4-triazole according to Claim 1, in which R1 is a C1-C4 alkyl group substituted with 1 to 3 hydroxyl radicals and R2 is a phenyl group which may be substituted with 1 to 3 radicals.

3. Pyrazolo[3,2-c]-1,2,4-triazole according to Claim 2, in which R1 is chosen from hydroxymethyl, 2-hydroxyethyl, 3-hydroxypropyl, 1-hydroxyethyl, 1-hydroxypropyl, 1-methyl-2-hydroxyethyl, 1,2-dihydroxyethyl, 2,3-dihydroxypropyl, 1,3-dihydroxypropyl, 1,3-dihydroxybutyl, 2,4-dihydroxybutyl, 1,2,3-trihydroxypropyl, 1,2,3-trihydroxybutyl.

4. Pyrazolo[3,2-c]-1,2,4-triazole according to Claim 2, in which R2 is chosen from a phenyl group or a phenyl group substituted with one or two radicals chosen from halogen, C1-C4 alkyl, C1-C4 alkoxy, hydroxyl, carboxyl, C1-C4 alkylthio, amino, C1-C4 alkylamino, C1-C4 dialkylamino and methylenedioxy radicals.

5. Pyrazolo[3,2-c]-1,2,4-triazole according to any one of the preceding claims, in which the addition salts with an acid are chosen from hydrochlorides, hydrobromides, sulphates, tartrates, benzenesulphonates, lactates, tosylates and acetates, and the addition salts with a base are chosen from the addition salts with sodium hydroxide, potassium hydroxide, aqueous ammonia, amines or alkanolamines.

6. Pyrazolo[3,2-c]-1,2,4-triazole according to any one of the preceding claims, chosen from the following compounds or their addition salts with an acid or a base:
6-hydroxymethyl-3-phenylpyrazolo[3,2-c]-1,2,4-triazole
6-hydroxymethyl-3-(p-toluyl)pyrazolo[3,2-c]-1,2,4-triazole
6-hydroxymethyl-3-(o-toluyl)pyrazolo[3,2-c]-1,2,4-triazole
6-hydroxymethyl-3-(m-toluyl)pyrazolo[3,2-c]-1,2,4-triazole
6-hydroxymethyl-3-(3-hydroxyphenyl)pyrazolo[3,2-c]-1,2,4-triazole
6-hydroxymethyl-3-(4-hydroxyphenyl)pyrazolo[3,2-c]-1,2,4-triazole
6-hydroxymethyl-3-(3-aminophenyl)pyrazolo[3,2-c]-1,2,4-triazole
6-hydroxymethyl-3-(4-aminophenyl)pyrazolo[3,2-c]-1,2,4-triazole
6-(2-hydroxyethyl)-3-phenylpyrazolo[3,2-c]-1,2,4-triazole
6-(2-hydroxyethyl)-3-(p-toluyl)pyrazolo[3,2-c]-1,2,4-triazole
6-(2-hydroxyethyl)-3-(o-toluyl)pyrazolo[3,2-c]-1,2,4-triazole
6-(2-hydroxyethyl)-3-(m-toluyl)pyrazolo[3,2-c]-1,2,4-triazole
6-(2-hydroxyethyl)-3-(3-hydroxyphenyl)pyrazolo[3,2-c]-1,2,4-triazole
6-(2-hydroxyethyl)-3-(4-hydroxyphenyl)pyrazolo[3,2-c]-1,2,4-triazole
*6*-(2-hydroxyethyl)-3-(3-aminophenyl)pyrazolo[3,2-c]-1,2,4-triazole
6-(2-hydroxyethyl)-3-(4-aminophenyl)pyrazolo[3,2-c]-1,2,4-triazole
6-(1-hydroxyethyl)-3-phenylpyrazolo[3,2-c]-1,2,4-triazole
6-(1-hydroxyethyl)-3-(p-toluyl)pyrazolo[3,2-c]-1,2,4-triazole
6-(1-hydroxyethyl)-3-(o-toluyl)pyrazolo[3,2-c]-1,2,4-triazole
6-(1-hydroxyethyl)-3-(m-toluyl)pyrazolo[3,2-c]-1,2,4-triazole
6-(1-hydroxyethyl)-3-(3-hydroxyphenyl)pyrazolo[3,2-c]-1,2,4-triazole
6-(1-hydroxyethyl)-3-(4-hydroxyphenyl)pyrazolo[3,2-c]-1,2,4-triazole
6-(1-hydroxyethyl)-3-(3-aminophenyl)pyrazolo[3,2-c]-1,2,4-triazole
6-(1-hydroxyethyl)-3-(4-aminophenyl)pyrazolo[3,2-c]-1,2,4-triazole.

7. Pyrazolo[3,2-c]-1,2,4-triazole according to Claim 6, chosen from the following compounds or their addition salts with an acid or a base:
6-hydroxymethyl-3-phenylpyrazolo[3,2-c]-1,2,4-triazole
6-hydroxymethyl-3-(p-toluyl)pyrazolo[3,2-c]-1,2,4-triazole
6-hydroxymethyl-3-(o-toluyl)pyrazolo[3,2-c]-1,2,4-triazole
6-hydroxymethyl-3-(m-toluyl)pyrazolo[3,2-c]-1,2,4-triazole
6-hydroxymethyl-3-(3-hydroxyphenyl)pyrazolo[3,2-c]-1,2,4-triazole
6-hydroxymethyl-3-(4-hydroxyphenyl)pyrazolo[3,2-c]-1,2,4-triazole
6-hydroxymethyl-3-(3-aminophenyl)pyrazolo[3,2-c]-1,2,4-triazole
6-hydroxymethyl-3-(4-aminophenyl)pyrazolo[3,2-c]-1,2,4-triazole.

8. Composition for dyeing keratinous fibres, and in particular human keratinous fibres such as hair, comprising, in an appropriate dyeing medium, at least one pyrazolo[3,2-c]-1,2,4-triazole coupler of formula (I) or one of its addition salts with an acid or a base as defined according to any one of Claims 1 to 7 and at least one oxidation base.

9. Composition according to Claim 8, in which the pyrazolotriazole coupler is such that R1 is a C1-C4 alkyl group substituted with 1 to 3 hydroxyl radicals and R2 is a phenyl group which may be substituted with 1 to 3 radicals.

10. Composition according to either of Claims 8 and 9, in which the pyrazolo-t-[3,2-c]-1,2,4-triazole coupler(s) of formula (I) are present in a quantity of between 0.001 and 10%, preferably between 0.005 and 6%.

11. Composition according to any one of Claims 8 to 10, in which the oxidation base(s) are chosen from para-phenylenediamines, bis-phenylalkylenediamines, para-aminophenols, ortho-aminophenols, heterocyclic bases, and their addition salts with an acid.

12. Composition according to any one of Claims 8 to 11, in which the oxidation base(s) are present in a quantity of between 0.001 and 10%, preferably between 0.005 and 6%.

13. Composition according to any one of Claims 8 to 12, further comprising a direct dye.

14. Method for the oxidation dyeing of keratinous fibres, and in particular human keratinous fibres such as hair, **characterized in that** at least one composition as defined in any one of Claims 8 to 13 is applied to the fibres, and **in that** the colour is developed with the aid of an oxidizing agent.

15. Method according to Claim 14, in which the oxidizing agent is chosen from hydrogen peroxide, urea peroxide, alkali metal bromates, persalts such as perborates and persulphates, peracids and oxidase enzymes.

16. Method according to Claim 14 or 15, in which the oxidizing agent is mixed at the time of use with the composition as defined according to any one of Claims 8 to 13.

17. Method according to either of Claims 14 and 15, in which the oxidizing agent is applied in the form of an oxidizing composition simultaneously with or sequentially to the composition as defined according to any one of Claims 6 to 13 to the fibres.

18. Multicompartment device or multicompartment dyeing "kit", in which a first compartment contains a composition as defined in any one of Claims 8 to 13 and a second compartment contains an oxidizing composition.

19. Coloured product which can be obtained by reacting at least one pyrazolo--[3,2-c]-1,2,4-triazole coupler of formula (I) or one of its addition salts with an acid or a base as defined according to any one of Claims 1 to 7, and at least one oxidation base.

20. Method for preparing a compound of formula (II) 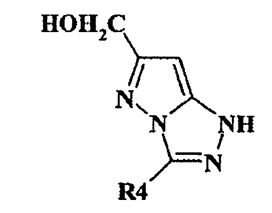 from a compound of formula (III) 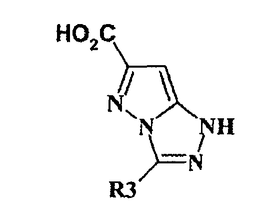 in which formulae R3 and R4, which are identical or different, represent an aryl group which may be substituted with at least one radical chosen from the group consisting of halogen, alkyl, hydroxyl, alkoxy, amino, alkylamino, dialkylamino and alkylthio radicals, the method comprising a step of reducing the carboxyl functional group of the compound (III) to an alcohol functional group.

21. Method according to Claim 20, comprising one or more intermediate steps aimed at converting R3 to R4, when R3 is different from R4.

22. Method according to Claim 20 or 21, in which the carboxylic acid functional group of the compound (III) is converted to the ester functional group before the reducing step.

## Patentansprüche

1. Pyrazolo-[3,2-c]-1,2,4-triazol der Formel (I) oder eines seiner Additionssalze mit einer Säure oder einer Base: 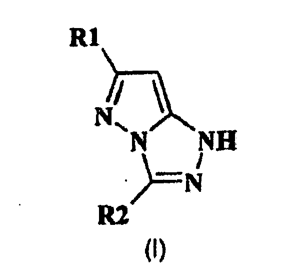 worin bedeuten:
R1 eine Hydroxygruppe oder eine C1-4-Alkylgruppe, die mit mindestens einer Hydroxygruppe substituiert ist, und
R2 eine Arylgruppe, die mit mindestens einer Gruppe substituiert sein kann, die unter den Gruppen Halogen, Nitro, Cyano, Alkyl, Hydroxy, Alkoxy, Amino, Alkylamino, Dialkylamino, Acylamino, Sulfonamido, Alkylthio, Alkoxycarbonyl, Carboxy und Sulfo ausgewählt ist.

2. Pyrazolo-[3,2-c]-1,2,4-triazol nach Anspruch 1, wobei R1 eine mit 1 bis 3 Hydroxygruppen substituierte C₁₋₄-Alkylgruppe ist und R2 eine Phenylgruppe bedeutet, die mit 1 bis 3 Gruppen substituiert sein kann.

3. Pyrazolo-[3,2-c]-1,2,4-triazol nach Anspruch 2, wobei R1 unter Hydroxymethyl, 2-Hydroxyethyl, 3-Hydroxypropyl, 1-Hydroxyethyl, 1-Hydroxypropyl, 1-Methyl-2-hydroxyethyl, 1,2-Dihydroxyethyl, 2,3-Dihydroxypropyl, 1,3-Dihydroxypropyl, 1,3-Dihydroxybutyl, 2,4-Dihydroxybutyl, 1,2,3-Trihydroxypropyl, 1,2,3-Trihydroxybutyl ausgewählt ist.

4. Pyrazolo-[3,2-c]-1,2,4-triazol nach Anspruch 2, wobei R2 unter Phenyl, Phenylgruppen, die mit ein oder zwei Gruppen substituiert sind, die unter Halogen, C₁₋₄-Alkyl, C₁₋₄-Alkyl, C₁₋₄-Alkoxy, Hydroxy, Carboxy, C₁₋₄-Alkylthio, Amino, C₁₋₄-Alkylamino, C₁₋₄₋Dialkylamino und Methylendioxy ausgewählt sind.

5. Pyrazolo-[3,2-c]-1,2,4-triazol nach einem der vorhergehenden Ansprüche, wobei die Additionssalze mit einer Säure unter den Hydrochloriden, Hydrobromiden, Sulfaten, Tartraten, Benzolsulfonaten, Lactaten, Tosylaten und Acetaten ausgewählt sind, und die Additionssalze mit einer Base unter den Additionssalzen mit Natriumhydroxid, Kaliumhydroxid, Ammoniak, Amino oder Alkanolaminen ausgewählt sind.

6. Pyrazolo-[3,2-c]-1,2,4-triazol nach einem der vorhergehenden Ansprüche, das unter den folgenden Verbindungen oder deren Additionssalzen mit einer Säure oder einer Base ausgewählt ist:
6-Hydroxymethyl-3-phenyl-pyrazolo [3, 2-c]-1,2,4-triazol
6-Hydroxymethyl-3-(p-toluyl)-pyrazolo[3,2-c]-1,2,4-triazol
6-Hydroxymethyl-3-(o-toluyl)-pyrazolo[3,2-c]-1,2,4-triazol
6-Hydroxymethyl-3-(m-toluyl)-pyrazolo[3,2-c]-1,2,4-triazol
6-Hydroxymethyl-3-(3-hydroxyphenyl)-pyrazolo[3,2-c]-1,2,4-triazol
6-Hydroxymethyl-3-(4-hydroxyphenyl)-pyrazolo[3,2-c]-1,2,4-triazol
6-Hydroxymethyl-3-(3-aminophenyl)-pyrazolo[3,2-c]-1,2,4-triazol
6-Hydroxymethyl-3-(4-aminophenyl)-pyrazolo[3,2-c]-1,2,4-triazol
6-(2-Hydroxyethyl)-3-phenyl-pyrazolo[3,2-c]-1,2,4-triazol
6-(2-Hydroxyethyl)-3-(p-toluyl)-pyrazolo[3,2-c]-1,2,4-triazol
6-(2-Hydroxyethyl)-3-(o-toluyl)-pyrazolo[3,2-c]-1,2,4-triazol
6-(2-Hydroxyethyl)-3-(m-toluyl)-pyrazolo[3,2-c]-1,2,4-triazol
6-(2-Hydroxyethyl)-3-(3-hydroxyphenyl)-pyrazolo[3,2-c]-1,2,4-triazol
6-(2-Hydroxyethyl)-3-(4-hydroxyphenyl)-pyrazolo[3,2-c]-1,2,4-triazol
6-(2-Hydroxyethyl)-3-(3-aminophenyl)-pyrazolo[3,2-c]-1,2,4-triazol
6-(2-Hydroxyethyl)-3-(4-aminophenyl)-pyrazolo[3, 2-c]-1,2,4-triazol
6-(1-Hydroxyethyl)-3-phenyl-pyrazolo[3,2-c]-1,2,4-triazol
6-(1-Hydroxyethyl)-3-(p-toluyl)-pyrazolo[3,2-c]-1,2,4-triazol
6-(1-Hydroxyethyl)-3-(o-toluyl)-pyrazolo[3,2-c]-1,2,4-triazol
6-(1-Hydroxyethyl)-3-(m-toluyl)-pyrazolo[3,2-c]-1,2,4-triazol
6-(1-Hydroxyethyl)-3-(3-hydroxyphenyl)-pyrazolo[3,2-c]-1,2,4-triazol
6-(1-Hydroxyethyl)-3-(4-hydroxyphenyl)-pyrazolo[3,2-c]-1,2,4-triazol
6-(1-Hydroxyethyl)-3-(3-aminophenyl)-pyrazolo[3,2-c]- 1,2,4-triazol
6-(1-Hydroxyethyl)-3-(4-aminophenyl)-pyrazolo[3,2-c]-1,2,4-triazol.

7. Pyrazolo-[3,2-c]-1,2,4-triazol nach Anspruch 6, das unter den folgenden Verbindungen oder deren Additionssalzen mit einer Säure oder mit einer Base ausgewählt ist:
6-Hydroxymethyl-3-phenyl-pyrazolo[3,2-c]-1,2,4-triazol
6-Hydroxymethyl-3-(*p*-toluyl)-pyrazolo[3,2-c]-1,2,4-triazol
6-Hydroxymethyl-3-(*o*-toluyl)-pyrazolo[3,2-c]-1,2,4-triazol
6-Hydroxymethyl-3-(m-toluyl)-pyrazolo[3,2-c]-1,2,4-triazol
6-Hydroxymethyl-3-(3-hydroxyphenyl)-pyrazolo[3,2-c]-1,2,4-triazol
6-Hydroxymethyl-3-(4-hydroxyphenyl)-pyrazolo[3,2-c]-1,2,4-triazol
6-Hydroxymethyl-3-(3-aminophenyl)-pyrazolo[3,2-c]-1,2,4-triazol
6-Hydroxymethyl-3-(4-aminophenyl)-pyrazolo[3,2-c]-1,2,4-triazol.

8. Zusammensetzung zum Färben von Keratinfasern und insbesondere menschlichen Keratinfasern, wie Haaren, die in einem zum Färben geeigneten Medium mindestens einen Pyrazolo-[3,2-c]-1,2,4-triazol-Kuppler der Formel (I) oder eines seiner Additionssalze mit einer Säure oder einer Base nach einem der Ansprüche 1 bis 7 und mindestens eine Oxidationsbase enthält.

9. Zusammensetzung nach Anspruch wobei der Pyrazolo-triazol-Kuppler so vorliegt, dass R1 eine C₁₋₄-Alkyl Gruppe, die mit 1 bis 3 Hydroxygruppen substituiert ist, und R2 eine Phenylgruppe ist, die mit 1 bis 3 Gruppen substituiert sein kann.

10. Zusammensetzung nach einem der Ansprüche 8 oder 9, wobei der oder die Pyrazolo-[3,2-c]-1,2,4-triazol-Kuppler der Formel (I) in einer Menge von 0,001 bis 10 % und vorzugsweise 0,005 bis 6 % enthalten sind.

11. Zusammensetzung nach einem der Ansprüche 8 bis 10, wobei die Oxidationsbase(n) unter den *p*-Phenylendiaminen, Bisphenylalkylendiaminen, p-Aminophenolen, *o*-Aminophenolen, heterocyclischen Basen und deren Additionssalzen mit einer Säure ausgewählt ist.

12. Zusammensetzung nach einem der Ansprüche 8 bis 11, wobei die Oxidationsbase(n) in einer Menge von 0,001 bis 10 % und vorzugsweise 0,005 bis 6 % enthalten ist.

13. Zusammensetzung nach einem der Ansprüche 8 bis 12, die ferner einen Direktfarbstoff enthält.

14. Verfahren zum oxidativen Färben von Keratinfasern und insbesondere menschlichen Keratinfasern wie Haaren, **dadurch gekennzeichnet, dass** auf die Fasern mindestens eine Zusammensetzung nach einem der Ansprüche 8 bis 13 aufgetragen und die Farbe mit einem Oxidationsmittel gebildet wird.

15. Verfahren nach Anspruch 14, wobei das Oxidationsmittel unter Wasserstoffperoxid, Harnstoffperoxid, Alkalimetallbromaten, Salzen von Persäuren, wie Perboraten und Persulfaten, Persäuren und Oxidasen ausgewählt ist.

16. Verfahren nach einem der Ansprüche 14 bis 15, wobei das Oxidationsmittel bei der Anwendung mit der Zusammensetzung nach einem der Ansprüche 8 bis 13 vermischt wird.

17. Verfahren nach einem der Ansprüche 14 oder 15, wobei das Oxidationsmittel in Form einer oxidierenden Zusammensetzung gleichzeitig mit oder getrennt von der Zusammensetzung nach einem der Ansprüche 6 bis 13 auf die Fasern aufgetragen wird.

18. Vorrichtung mit mehreren Abteilungen oder Kit zum Färben mit mehreren Abteilungen, wobei eine erste Abteilung eine Zusammensetzung nach einem der Ansprüche 8 bis 13 und eine zweite Abteilung eine oxidierende Zusammensetzung enthält.

19. Farbiges Produkt, das durch Reaktion mindestens eines Pyrazolo-[3,2-c]-1,2,4-triazol-Kupplers der Formel (I) oder eines seiner Additionssalze mit einer Säure oder einer Base nach einem der Ansprüche 1 bis 7 und mindestens einer Oxidationsbase erhalten wird.

20. Verfahren zur Herstellung einer Verbindung der Formel (II) 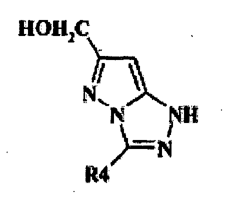 aus einer Verbindung der Formel (III) 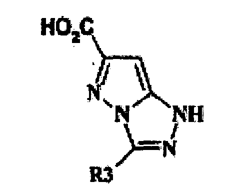 wobei in den Formeln R3 und R4, die gleich oder verschieden sind, eine Arylgruppe bedeuten, die mit mindestens einer Gruppe substituiert sein, die unter den Gruppen Halogen, Alkyl, Hydroxy, Alkoxy, Amino, Alkylamino, Dialkylamino und Alkylthio ausgewählt ist, wobei das Verfahren die Reduktion der Carboxyfunktion der Verbindung (III) in die Alkoholfunktion umfasst.

21. Verfahren nach Anspruch 20, das einen oder mehrere Zwischenschritte umfasst, die dazu dienen, R3 in R4 umzuwandeln, falls R3 von R4 verschieden ist.

22. Verfahren nach Anspruch 20 oder 21, wobei die Carbonsäurefunktion der Verbindung (III) vor der Reduktion in die Esterfunktion umgewandelt wird.
